# EUROPEAN PATENT APPLICATION

(11) **EP 1 426 871 A1**
(43) Date of publication of application: **09.06.2004**
(21) Application number: 03026724.9
(22) Date of filing: 21.11.2003
(51) Int. Cl.: G06F 17/00, A61B 5/055, G01R 33/48

(54) **Method and apparatus for analyzing brain functions**

(30) Priority: 27.11.2002 JP 2002343195
(71) Applicant: Communications Research Laboratory, Independent Administrative Institution, Koganei-City, Tokyo 184-0015 (JP)
(72) Inventor: Miyauchi, Satoru, Communications Res. Lab., Koganei-City Tokyo 184-0015 (JP)
(74) Representative: Müller - Hoffmann & Partner

(57) **Abstract**

An apparatus for analyzing brain functions is provided, which includes: biosignal detection means for detecting a biosignal of an examinee in parallel with examination of the brain of the examinee conducted by an MRI system; and a functioning part location calculating means for finding out a part of the brain functioning in a state where a predetermined event is occurring in the biosignal by calculation based on a correlation between time-series data of the biosignal and a change in a strength of a MRI signal outputted from the MRI system.

## Description

### BACKGROUND OF THE INVENTION

### Field of the Invention

The present invention relates to method and apparatus for analyzing brain functions by using an MRI system.

### Description of the Related Art

BOLD contrast, which is a representative analyzing principle of fMRI, detects variations in magnetization susceptibility effect with varying oxygen concentration in blood flowing in brain to determine an acting state of cranial nerve cells by using an MRI system. Specifically, BOLD contrast finds out the correlativity between cycles of paradigm (task to be fulfilled) imposed on an examinee and a change in MRI signal strength with varying distribution of blood kinetics in a localized body part. The correlativity thus found out is detected as an indirect expression of nerve excitation.

More specifically, fMRI causes an MRI system to perform tomography successively at fixed time intervals while having an examinee fulfill a certain task (watching a picture, listening to a sound, doing finger exercise, answering a question on words, or the like), as described in Japanese Patent Laid-Open Publication No. HEI 09-117430. The relationship between the fulfillment of the task and the signal strength at one pixel or voxel forming an image is compared to that between the fulfillment of the task and the signal strength at another pixel or voxel. A part at which the signal strength varies in relation to the task is found to have a correlation with a reference function representing the task, whereas a part not related to the task is detected as a mere noise. Whether the task and the change in signal strength have a relationship therebetween is judged based on statistic significance.

However, such a method in which a task is imposed on an examinee is known to have a difficulty in analysing the functions of certain parts of brain including thalamus, putamen and pons. Recently, such parts are being considered to participate in memory processing during sleeping. However, verification of this consideration is difficult because actions of such parts are difficult to analyze. Though there was a case where brain functions during sleeping were analyzed by employing a PET system, this case did not intend to analyze brain functions related to memory processing. Further, since such a PET system has a very low time resolution as compared to the MRI system, it is difficult for the PET system to analyze brain functions related to memory processing for each of minutely divided sleeping stages.

With fMRI, the examinee must fulfill the task without moving his or her head because measurement errors occur if the examinee moves his or her head in the MRI system during examination. For this reason, fMRI sometimes imposes a heavy burden on the examinee. Further, it may be impossible for an examinee having some disorder of a brain function to fulfill such a task.

Accordingly, it is an object of the present invention to provide a method and apparatus for analyzing brain functions, which are capable of reducing the burden on an examinee as well as analyzing a function of a part of brain which cannot be analyzed by the prior art thereby providing novel possibilities related to the study of brain, for example, the study of memory processing of brain. The method and apparatus of the present invention utilize biosignal detection means which is capable of detecting a biosignal of the examinee in parallel with and separately from examination conducted by an MRI system and conduct analysis of parts of brain that are functioning based on a correlation between time-series data of the biosignal and a change in MRI signal strength, whereby brain functions can be analyzed with the examinee not requested to fulfill any task, for example, with the examinee requested only to sleep.

### SUMMARY OF THE INVENTION

In accordance with the present invention, there is provided an apparatus for analyzing brain functions, comprising: biosignal detection means for detecting a biosignal of an examinee in parallel with examination of the brain of the examinee conducted by an MRI system; and a functioning part location calculating means for finding out a part of the brain functioning in a state where a predetermined event is occurring in the biosignal by calculation based on a correlation between time-series data of the biosignal and a change in a strength of a MRI signal outputted from the MRI system. The expression "in parallel with", as used herein, is meant to include a concept of "substantially simultaneously with".

The apparatus thus constructed is capable of analyzing brain functions based only on a biosignal obtained from an examinee who is requested only to rest stationary (to sleep for example). Since the apparatus need not impose any task on the examinee from outside, the apparatus is capable of largely reducing the burden on the examinee during analysis as well as conducting brain function analysis on the examinee with ease even if he or she has a malady or some disorder in his or her brain.

Preferably, the predetermined event is an event based on which a waking level of the examinee is identified.

Preferably, the biosignal detection means is configured to detect an electroencephalogram of the examinee as the biosignal.

Preferably, the detection of the biosignal of the examinee by the biosignal detection means and the examination of the brain of the examinee by the MRI system are performed alternately.

If the apparatus is capable of detecting an electroencephalogram of an examinee or the like as the biosignal to identify the current waking level of the examinee, the apparatus makes it possible to analyze actions of specific parts of brain, including thalamus, putamen and the like, which have heretofore been considered difficult to analyze. Since such specific parts of brain are said to be closely related to memory processing, the apparatus of the present invention can provide novel possibilities in objective diagnosis of disturbance of memory, senile dementia of Alzheimer type, Parkinson disease or the like, as well as in elucidation of the memory mechanism.

In accordance with the present invention, there is also provided a method of analyzing brain functions, comprising the steps of: detecting a biosignal of an examinee in parallel with examination of the brain of the examinee conducted by an MRI system; and finding out a part of the brain functioning in a state where a predetermined event is occurring in the biosignal by calculation based on a correlation between time-series data of the biosignal and a change in a strength of a MRI signal outputted from the MRI system.

The foregoing and other objects, features and attendant advantages of the present invention will become apparent from the reading of the following detailed description in conjunction with the accompanying drawings.

### BRIEF DESCRIPTION OF THE DRAWINGS

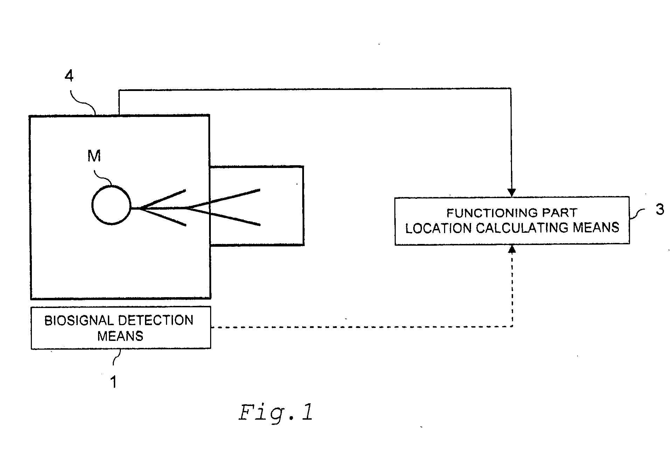

Fig. 1 is a diagram schematically showing the overall construction of the present invention;
Fig. 2 is a schematic block diagram showing the overall configuration of a brain function analyzing apparatus according to one embodiment of the present invention;
Fig. 3 is a chart of time-series data on an EEG signal superimposed with noise in the same embodiment;
Fig. 4 is a chart of time-series data on a shaped EEG signal in the same embodiment;
Fig. 5 is a chart of a power spectrum within a predetermined time period obtained from analysis of an EEG signal in the same embodiment;
Fig. 6 is a chart of time-series data on the frequency of occurrences of electroencephalogram for each frequency band in the same embodiment;
Fig. 7 is a chart of time-series data on the frequency of occurrences of electroencephalogram for each frequency band in the same embodiment;
Fig. 8 is a flowchart of an operation of the brain function analyzing apparatus according to the same embodiment;
Fig. 9 is a flowchart of an operation of the brain function analyzing apparatus according to the same embodiment;
Fig. 10 is a flowchart of an operation of the brain function analyzing apparatus according to the same embodiment; and
Fig. 11 is a graphic representation of exemplary images of functioning states of brain obtained by the brain function analyzing apparatus according to the same embodiment.

### DETAILED DESCRIPTION OF THE PREFERRED EMBODIMENTS

The present invention will now be described in detail by way of a preferred embodiment thereof with reference to the drawings.

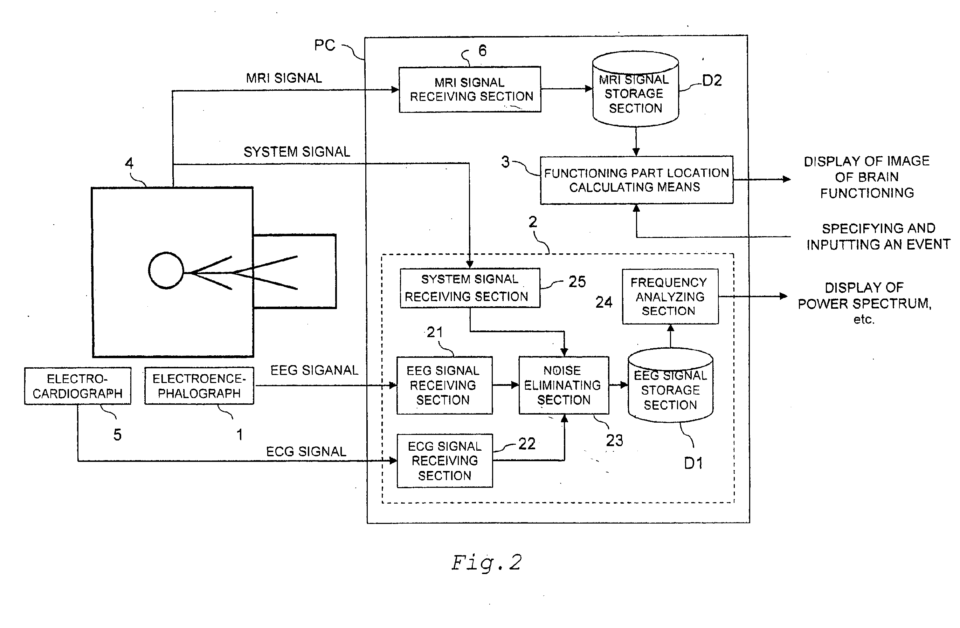

Referring first to Fig. 2, a brain function analyzing apparatus according to this embodiment includes, at least, biosignal detection means 1 for detecting a biosignal of an examinee M in parallel with examination of the brain of the examinee M conducted by an MRI system 4 and outputting the same, event identification support means 2 for supporting identification of an event occurring in the living body of the examinee M from time-series data on the biosignal, and a functioning part location calculating means 3 for finding out a part of the brain functioning in a state where a predetermined event is occurring by calculation based on a correlation between the time-series data on the biosignal and a change in the strength of a MRI signal outputted from the MRI system 4. In this embodiment an information processor PC comprising a CPU, a storage unit and the like, such as a personal computer, functions as the event identification support means 2 and the functioning part location calculating means 3.

Each part of the apparatus will be described below.

The MRI system 4 is configured to image an internal sectional structure of the examinee M exposed to a strong magnetic field by causing atomic nuclei of hydrogen forming part of water and fat to resonate by application of radio wave. The MRI system 4 used in this embodiment divides brain into 4×4×5mm voxels by one-time scanning (about 4 seconds) and outputs MRI signals from respective voxels. One scanning cycle is completed in about 8 seconds comprising a scanning period of about 4 seconds and a scanning halted period of about 4 seconds following the scanning period. The MRI system 4 is set to repeat the scanning cycle 500 times (in about 67 minutes).

The biosignal detection means 1 comprises an electroencephalograph for example and is adapted to detect an electroencephalogram and then output it as an EEG signal. This embodiment further comprises an electrocardiograph 5 and utilizes an ECG signal outputted therefrom in order to eliminate the influence of heart beat superimposed on the EEG signal.

The event identification support means 2 comprises an EEG signal receiving section 21 for receiving the EEG signal, an ECG signal receiving section 22 for receiving the ECG signal, system signal receiving section 5 for receiving a signal generated from the MRI system 4, a noise eliminating section 23 for eliminating heart beat noise and system noise generated accompanying the operation of the MRI system 4, which are superimposed on the EEG signal, and a frequency analyzing section 24 for analyzing the frequency of the EEG signal from which the aforementioned noises have been eliminated and outputting the result of analysis in various forms.

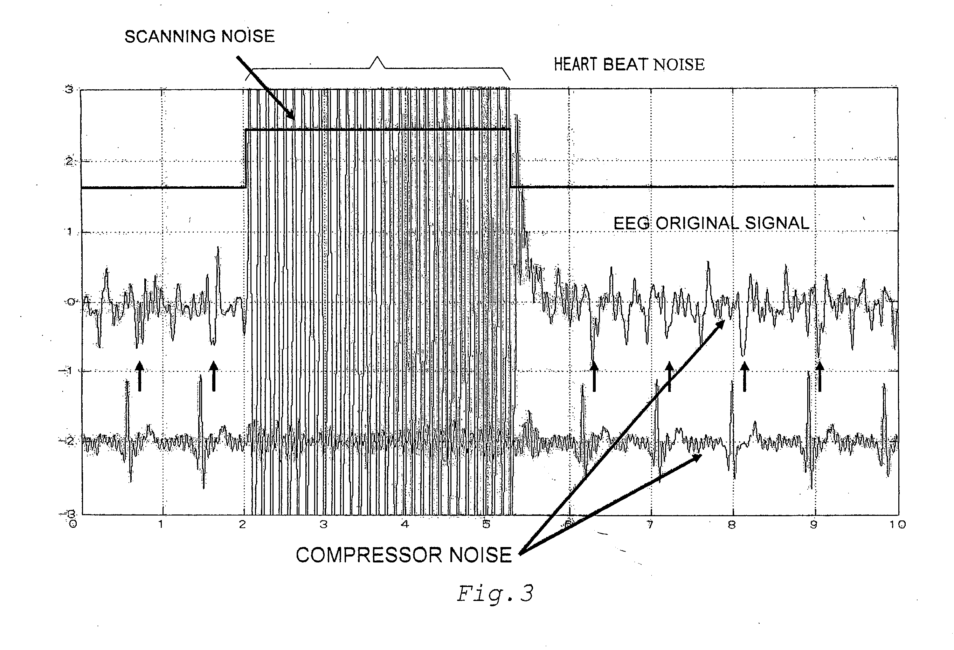

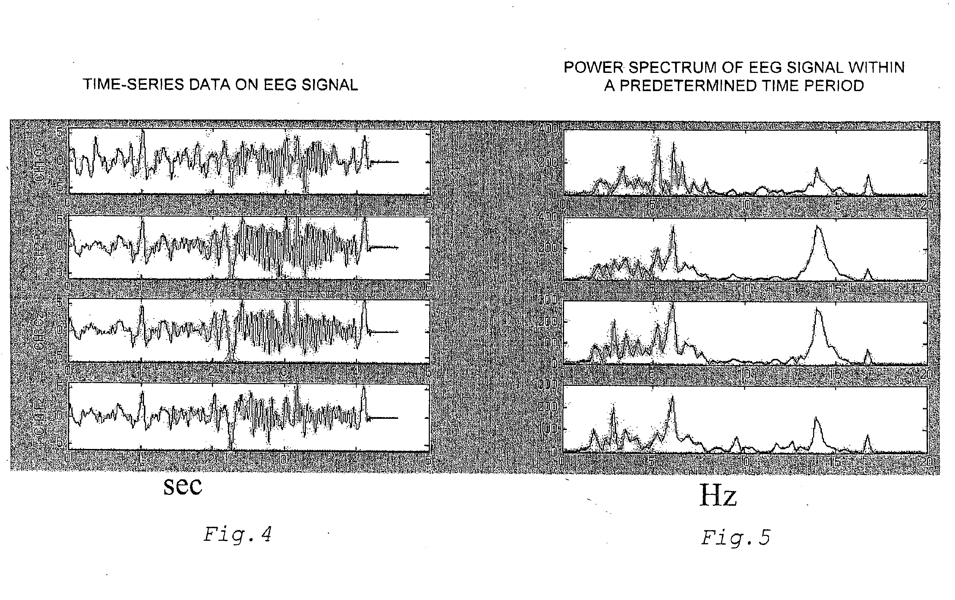

Since a raw EEG signal (EEG original signal) contains heat beat noise and compressor noise as superimposed as shown in Fig. 3, the aforementioned noise eliminating section 23 is configured to subtract a heart beat noise pattern and a compressor noise pattern, which are generated by averaging a multiplicity of such EEG original signals previously added to each other, from the EEG original signal by causing the noise patterns to synchronize respective of the ECG signal and compressor operation signal received, thereby eliminating the influence thereof. Further, the noise eliminating section 23 has a function of making effective only an EEG signal generated in a scanning halted period by neglecting an EEG signal generated during scanning based on a scanning signal generated from the MRI system 4. This is because such an EEG signal generated during scanning is of no use due to noise caused by induced electromotive force of a strong magnetic field generated during scanning. Consequently, detection of an electroencephalogram and examination by the MRI system 4 appear to be performed alternately (at time intervals of 4 seconds). Fig. 4 shows exemplary time-series data on an EEG signal thus shaped by the noise eliminating section 23. Since the electroencephalograph 1 has four channels, four pieces of data are shown in Fig. 4.

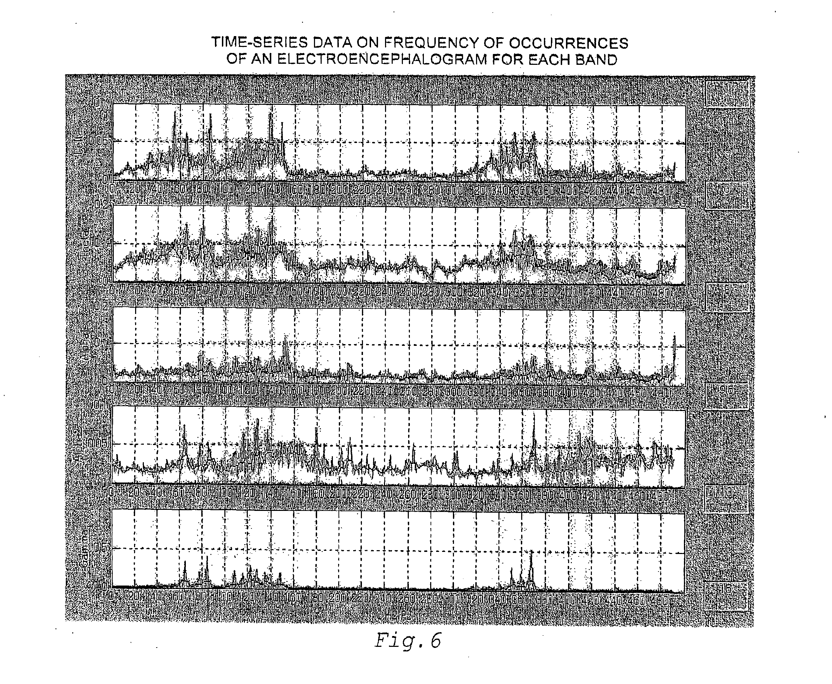

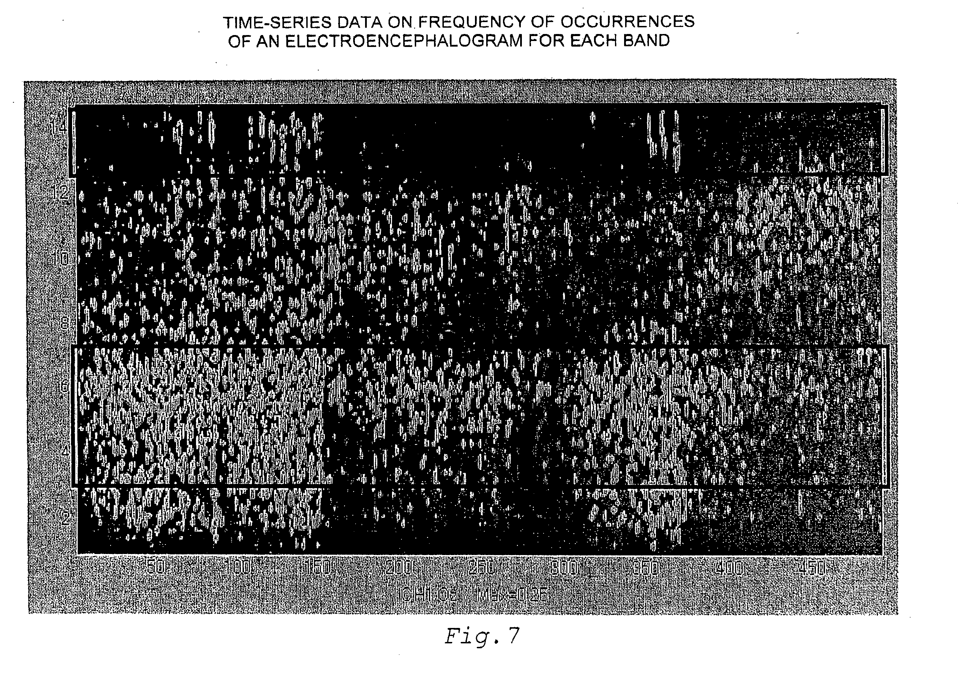

The aforementioned frequency analyzing section 24 analyzes, based on FFT, the frequency of the EEG signal that has been shaped by elimination of noise and the like by the noise eliminating section 23 and then outputs a display of a power spectrum using a frequency in a predetermined time slot as a parameter as shown in Fig. 5 or a display of data on the frequency of occurrences of a wave of interest with time for each of predetermined bands as shown in Figs. 6 and 7. Such frequency analysis enables an event occurring in a living body to be identified. The "event", as used in this embodiment, means a wave of a frequency characteristic of an electroencephalogram, which is indicative of a certain waking level of the examinee M. For example, such waking levels are classified into the following stages: waking stage, sleeping stage 1 (hypnagogic period and initial light sleeping period), sleeping stage 2 (light sleeping period), sleeping stage 3 (medium-level sleeping period), sleeping stage 4 (deep sleeping period), and stage REM. Since a wave (event) characteristic of each stage appears, a current waking level can be identified from the output of the frequency analyzing section 24. For example, the sleeping stage 1 allows a slow wave having a frequency in the vicinity of 4 to 6 Hz, which does not appear in the waking stage, to appear. The sleeping stage 2 allows a spindle having a frequency in the vicinity of 12 to 14 Hz and a K-complex to appear. Such a current waking level may be identified either automatically or by a human.

The functioning part location calculating means 3 specifies a part of brain functioning in a state where a predetermined event is occurring by obtaining information on MRI signal strengths at each voxel in a state where a predetermined event specified by the operator is occurring (in the sleeping stage 1 for example) and in a state where no predetermined event is occurring (in the waking stage for example) and then taking a differential of a change in MRI signal strength at each voxel that is considered significant in view of the correlativity with the change in state.

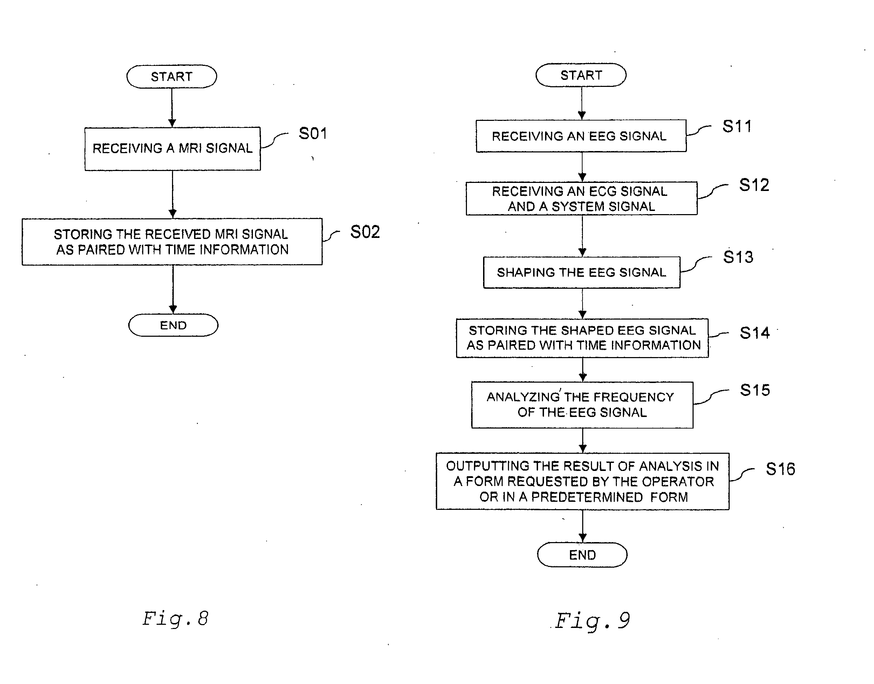

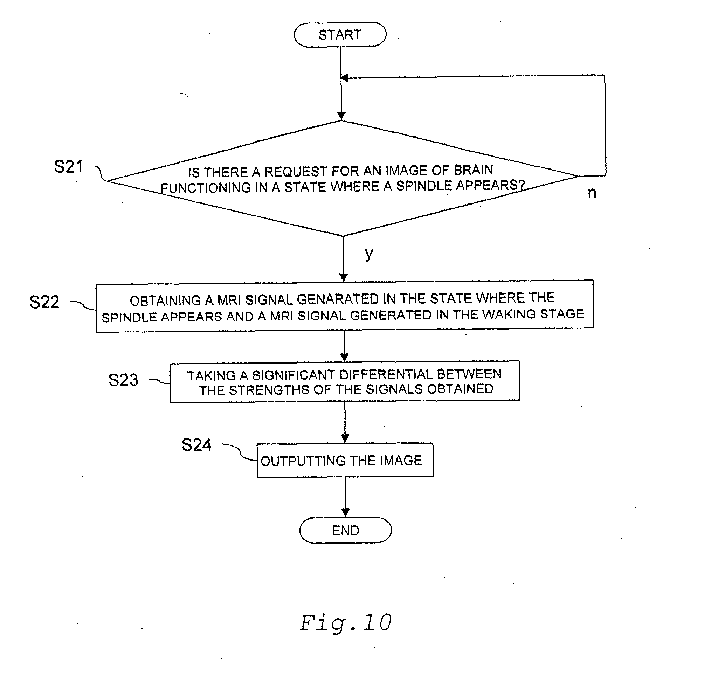

Referring to Figs. 8 to 10, description will be made of the operation of the brain function analyzing apparatus.

First, the examinee M fitted with the electroencephalograph is allowed to sleep in the MRI system 4. In the process of becoming asleep from the waking state, the examinee M is subjected to scanning of a predetermined duration (4 seconds) 500 times with predetermined cycles (8 seconds) by the MRI system 4. MRI signals generated by such scanning are received by the MRI signal receiving section 6 of the information processor PC (step S01) and then stored as paired with time information in a predetermined memory area (MRI signal storage section D2) (step S02).

On the other hand, an electroencephalogram of the examinee M detected by the electroencephalograph is continuously transmitted as an EEG signal to the information processor PC. At the same time, the electrocardiograph 5 transmits an ECG signal representing a heat beat wave to the information processor PC.

Thus, the information processor PC receives the EEG signal, ECG signal and a system signal of the MRI system 4 at the EEG signal receiving section 21, ECG signal receiving section 22 and system signal receiving section 5, respectively (steps S11 and S12). Then, the noise eliminating section 23 shapes the EEG signal by eliminating heart beat noise superimposed on the EEG signal and system noise generated accompanying the operation of the MRI system 4 while neglecting an EEG signal generated during scanning (step S13). The EEG signal thus shaped is stored as paired with time information in a predetermined memory area (EEG signal storage section D1) (step S14). Thereafter, the frequency analyzing section 24 analyzes the frequency of the EEG signal from which noises have been eliminated (step S15) and then outputs the result of the analysis in any one of various forms requested by the operator (for example, in a graphic form) (step S16).

When the operator having seen the outputted display requests outputting of an image of the brain functioning in a state where, for example, a spindle appears (in the sleeping stage 2) (step S21), the functioning part location calculating section 3 receives the request and then obtains information on the MRI signal strength at each voxel in that state from the MRI signal storage section D2 while obtaining information on the MRI signal strength at each voxel in a state where no predetermined event is occurring (in the waking stage for example) from the MRI signal storage section D2 also (step S22). Subsequently, the functioning part location calculating section 3 takes a differential of a change in MRI signal strength at each voxel that is considered significant in view of the correlativity with the change in state (step S23), specifies the part of brain that functions in the state where the spindle appears and outputs the image thereof (step S24).

An exemplary result of analysis conducted by the brain function analyzing apparatus is as follows.

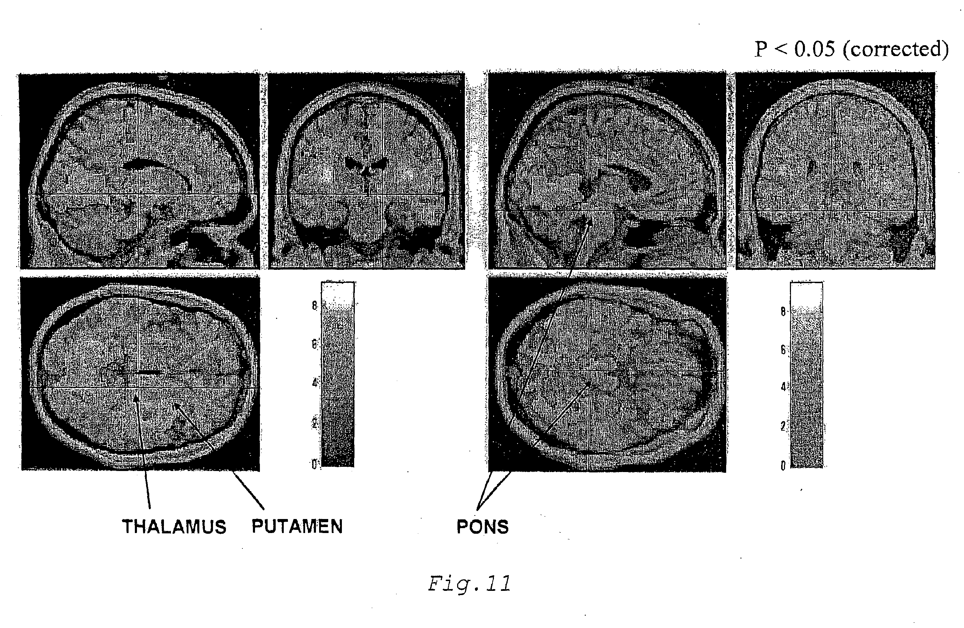

Fig. 11 shows images of parts of brain functioning in a state where θ-wave holds superiority over other waves and a spindle appears (in the sleeping stage 2) in comparison with images of the same parts in the waking stage. As can be seen from Fig. 11, the brain is found to be acting at thalamus, pons, putamen in basal ganglia, and the like. Such parts are said to have close relation to memory processing and their actions in the waking stage are very difficult to analyze. Recently, there has been set up the hypothesis that experience in the' waking stage is processed in brain and memorized as engram. The fact that the parts of brain related to memory processing were found to act briskly in the sleeping stage 2 is very interesting in that the fact is in line with the hypothesis.

The brain function analyzing apparatus according to this embodiment is capable of analyzing brain functions of the examinee M who is requested only to sleep. Since the apparatus need not impose any task on the examinee M, the apparatus is capable of largely reducing the burden on the examinee M during analysis as well as of conducting brain function analysis on the examinee M with ease even if he or she has, for example, a malady or some disorder in his or her brain.

Further, the apparatus makes it possible to analyze actions of specific parts of brain, including thalamus and putamen, which have heretofore been considered difficult to analyze. Since such specific parts are said to be closely related to memory processing, the apparatus according to this embodiment can provide novel possibilities in objective diagnosis of disturbance of memory, senile dementia of Alzheimer type, Parkinson disease or the like as well as in elucidation of the memory mechanism.

The present invention is not limited to the foregoing embodiment. Though the foregoing embodiment is configured to detect an electroencephalogram as a biosignal, the present invention may detect any one of other biosignals. For example, an electromyogram (EMG) signal and an electrooculogram (EOG) signal may be used either alone or in combination. By so doing, it is possible to recognize a change in the state of a living body in a more fragmented fashion thereby to elucidate a change in brain function in more detail accordingly.

While scanning by the MRI system 4 and electroencephalogram detection are conducted alternately in the foregoing embodiment, such scanning and electroencephalogram detection may be conducted simultaneously if noises superimposed on an EEG signal generated during scanning can be cancelled.

Further, the apparatus of the present invention need not necessarily be provided with event identification support means 2. Without even identification support means 2, the apparatus may be configured to allow the operator to specify an event and output an image of brain functioning in a state where the event specified by the operator is occurring in a substantially full automatic fashion if only the event is specified.

While only certain presently preferred embodiments of the present invention have been described in detail, as will be apparent for those skilled in the art, certain changes and modifications may be made in embodiments without departing from the spirit and scope of the present invention as defined by the following claims.

## Claims

1. An apparatus for analyzing brain functions, comprising: biosignal detection means for detecting a biosignal of an examinee in parallel with examination of the brain of the examinee conducted by an MRI system; and a functioning part location calculating means for finding out a part of the brain functioning in a state where a predetermined event is occurring in the biosignal by calculation based on a correlation between time-series data of the biosignal and a change in a strength of a MRI signal outputted from the MRI system.

2. The apparatus in accordance with claim 1, wherein the predetermined event is an event based on which a waking level of the examinee is identified.

3. The apparatus in accordance with claim 1 or 2, wherein the biosignal detection means is configured to detect an electroencephalogram of the examinee as the biosignal.

4. The apparatus in accordance with any one of claims 1 to 3, wherein the detection of the biosignal of the examinee by the biosignal detection means and the examination of the brain of the examinee by the MRI system are performed alternately.

5. A method of analyzing brain functions, comprising the steps of: detecting a biosignal of an examinee in parallel with examination of the brain of the examinee conducted by an MRI system; and finding out a part of the brain functioning in a state where a predetermined event is occurring in the biosignal by calculation based on a correlation between time-series data of the biosignal and a change in a strength of a MRI signal outputted from the MRI system.
